# EUROPÄISCHE PATENTANMELDUNG

(11) **EP 2 286 767 A1**
(43) Veröffentlichungstag der Anmeldung: **23.02.2011**
(21) Anmeldenummer: 09168354.0
(22) Anmeldetag: 21.08.2009
(51) Int. Cl.: A61F 2/30

(54) **Verfahren zur Herstellung einer strukturierten Oberfläche an einem Werkstück aus einem metallischen Werkstoff und Implantat mit einer derartigen Oberfläche**

(71) Anmelder: Jossi Holding AG, 8546 Islikon (CH)
(72) Erfinder: Gugler, Christian, 8500, Frauenfeld (CH); Nadler, Daniel, 8442, Hettlingen (CH); Casutt, Simon, 9200, Gossau (CH)
(74) Vertreter: Hepp, Dieter

(57) **Zusammenfassung**

An einem Werkstück (2) aus einem metallischen Werkstoff, insbesondere an einem Implantat, wird eine strukturierte Oberfläche (1) erzeugt, wobei diese mit einer Mehrzahl von sich gegen das innere des Werkstücks erweiternde Kavitäten (3) versehen ist. Diese weisen zur Oberfläche hin separate Öffnungen 4 auf und sie sind zur Bildung einer Gewölbestruktur (5) unter der Oberfläche miteinander verbunden. Die Kavitäten werden dabei durch ein elektrochemisches Abtragverfahren erzeugt. Vorzugsweise sind dabei benachbarte Kavitäten (3) durch spitzwinklige Kanten (9) begrenzt.

## Beschreibung

Die Erfindung betrifft ein Verfahren zur Herstellung einer strukturierten Oberfläche an einem Werkstück aus einem metallischen Werkstoff, insbesondere an einem Implantat, gemäss dem Oberbegriff von Anspruch 1, sowie ein Implantat aus einem metallischen Werkstoff gemäss dem Oberbegriff von Anspruch 5. Angestrebt wird mit einer derartigen Oberfläche eine bessere Verankerung des Implantats durch Einwachsen von Knochen in die strukturierte Oberfläche.

Im medizinischen Bereich sind verschiedene Oberflächestrukturen für Implantate bekannt. So beschreibt beispielsweise die EP 0 417 034 ein Implantat, bei dem in einem bestimmten Rasterabstand geometrische Raumformen unter der Oberfläche bestehen, die einerseits jeweils eine Durchtrittsbohrung zur Oberfläche aufweisen und andererseits unter der zusammenhängenden Oberfläche mit benachbarten Raumformen ineinander übergehen. Die strukturierte Oberfläche wird hier mittels eines Hochdruckflüssigkeitsstrahls, dem abrasive Partikel beigemischt sind, hergestellt, wobei eine Struktur mit sanften, verrundeten Übergängen erzeugt wird.

Das beschriebene Herstellungsverfahren hat den Nachteil, dass die einzelnen Raumformen nur sequentiell hergestellt werden können und der Einsatz eines Mehrfachwerkzeugs nicht möglich ist. Ausserdem können Überreste der im Herstellungsverfahren benutzten abrasiven Partikel im Körper nachteilige Effekte haben. Nicht rotationssymmetrische Raumformen lassen sich mit diesem Verfahren nicht herstellen. Auch wirken durch den Flüssigkeitsdruck hohe Kräfte auf das Werkstück, was zu Verformungen führen kann. Dies kann bei der Herstellung unter anderem zu mangelnder Präzision führen. Aufgrund der Strömungsverhälntisse lassen sich bei diesem Verfahren nur sehr schwer symmetrische Durchbrüche zwischen den Raumformen erzeugen.

Die EP 0 965 312 beschreibt eine Endoprothese aus Metall die ohne Zement implantierbar ist. Die Oberfläche der Prothese verfügt zumindest teilweise über Nuten mit Hinterschnitt. Diese Strukturen werden mittels eines Hochdruckwasserstrahls hergestellt.

Durch Zugabe abrasiver Partikel lässt sich zudem eine mikrorauhe Oberfläche erzeugen.

Ein wesentlicher Nachteil dieser Struktur ist darin zu sehen, dass eine Verankerung der Prothese mit dem Knochen nur orthogonal und quer zur Nute erfolgt, jedoch nicht in deren Längsrichtung.

EP 0 800 802 beschreibt ein Metallimplantat mit einer strukturierten Oberfläche. Diese Oberflächenstruktur besteht aus nebeneinander angeordneten Kugelkalotten, deren Tiefe bis ein weniges über den Radius einer Halbkugel geht. Die Kalotten werden unter anderem durch ein elektrochemisches Abtragverfahren oder durch chemisches Ätzen hergestellt. Dabei müssen Bereiche, die nicht abgetragen werden sollen, mit einem Schutzlack geschützt werden.

Ein wesentlicher Nachteil des beschriebenen Verfahrens ist eine relativ lange Bearbeitungszeit der Oberfläche sowie die dazu nötigen Prozessschritte wie Beschichten, partielles Abtragen des Schutzlacks und Ätzen. Ausserdem ist die Wirktiefe dieses Verfahrens begrenzt. Ein Nachteil der beschriebenen Oberfläche besteht auch darin, dass zwar Knochengewebe in die Kalotten einwächst, jedoch aufgrund eines fehlenden Hinterschnitts der Knochen sich nicht richtig in die Oberfläche verankern kann.

Aus der GB 2 181 354 ist schliesslich bekannt geworden, dass mittels elektrochemischer Abtragverfahren verschiedenförmige Erhöhungen oder Noppen auf eine Implantatoberfläche aufgebracht werden können. Ein entscheidender Nachteil dieser Oberflächestruktur ist die Gefahr, dass Noppen beim Implantieren abbrechen. Die angestrebte Harmonisierung des Steifigkeitsunterschieds zwischen Metall und Knochen kann nur durch fein strukturierte Noppen erfolgen, was die Gefahr des Abbrechens zusätzlich erhöht.

Eine Aufgabe der vorliegenden Erfindung besteht daher darin, ein Verfahren der eingangs genannten Art zu schaffen, das die Herstellung von komplexen Raumformen ermöglicht, um damit optimale Voraussetzungen für das Einwachsen von Knochengewebe in die Oberfläche zu ermöglichen. Ausserdem soll die Fertigung möglichst mittels Mehrfachwerkzeugen erfolgen. Diese Aufgabe wird erfindungsgemäss mit einem Verfahren mit den Merkmalen des Anspruchs 1 gelöst.

Die Erfindung nutzt die Tatsache aus, dass sich durch Elektrochemisches Abtragen kontrolliert Strukturen in und unter einer Werkstückoberfläche herstellen lassen. Das an sich bekannte Verfahren ist auch als Elysieren oder "electrochemical machining (ECM)" bekannt. Dabei wird ein positiv polarisiertes Werkstück (Anode) durch ein negativ polarisiertes Werkzeug (Kathode) in einem Elektrolyt unter Anlegen einer Spannungsquelle elektrochemisch aufgelöst. Die unterschiedlichen Formen der Kavitäten werden dabei durch geschickte Wahl der Abtragszeit und/oder der benutzten Abtragsspannung erreicht. Beispielsweise wird eine Kathode mit konstanter Geschwindigkeit auf ein Werkstück gesenkt, wobei ein Sackloch gebildet wird. Danach kann die Kathode eine gewisse Zeit lang in einer bestimmten Tiefe des Werkstücks gehalten werden, wobei sich die Kavität an der Spitze der Kathode vergrössert. Wird die Kathode zusätzlich stellenweise mit einer Isolationsschicht überzogen, lässt sich der Materialabtrag räumlich kontrollieren. Bevorzugt wird die Kathode derart elektrisch isoliert, dass nur an der Spitze Material abgetragen wird.

Das erfindungsgemässe Verfahren eignet sich vorteilhaft für die Oberflächenbearbeitung von Implantaten, insbesondere für den menschlichen oder tierischen Bewegungsapparat. In bestimmten Fällen könnte das Verfahren aber auch zur Herstellung von Oberflächen an anderen Werkstücken eingesetzt werden. Denkbar wären beispielsweise Werkstücke, deren Oberflächen besonders intensiv gekühlt werden müssen, da durch die Gewölbestruktur eine besonders grosse Oberfläche entsteht, die gut durchlüftet werden kann. Ausserdem eignet sich das Verfahren auch zur Optimierung von Klebeflächen. Ersichtlicherweise hinterlässt das Verfahren keinerlei Kontamination am Werkstück, was namentlich bei Implantaten von grosser Bedeutung ist.

Bevorzugt werden die Kavitäten mit einem mehrere Kathoden umfassenden Mehrfachwerkzeug gleichzeitig erzeugt. Im Gegensatz zum eingangs erwähnten abrasiven Bohren lassen sich so ganze Flächenabschnitte gleichzeitig bearbeiten.

In einer bevorzugten Ausführungsform der Erfindung werden nach der Erzeugung der Kavitäten als Grobstruktur die Parameter des Abtragprozesses derart verändert, dass die Kavitäten eine mikrorauhe Oberfläche, insbesondere mit einer Oberflächenrauhigkeit im Bereich von 1 bis 15µm, bevorzugt im Bereich von 1,6 bis 9µm erhalten. Veränderbar sind beispielsweise elektrische Parameter wie Stromdichte, Polarität, Spannung, Impulsdauer- und Form. Weiter können auch der Spüldruck, die Vorschubgeschwindigkeit sowie die Parameter des Elektrolyten, wie chemische Zusammensetzung, Konzentration und Temperatur verändert werden. Beispielsweise kann das Aufrauen der Oberfläche durch geringe Stromdichte und nicht optimaler Spülung erzeugt werden. Dabei entsteht eine sehr raue, mit Spitzen bedeckte Oberfläche.

Solche Mikrorauhigkeiten ermöglichen Mikroverbindungen zwischen den Knochentrabekeln und der Materialoberfläche und erhöhen somit die Verankerungsstabilität. Die Knochenbildenden Zellen (Osteoblasten) setzen sich bevorzugt auf Kanten und Spitzen, so dass das Anwachsen von Knochenmaterial an eine aufgeraute Oberfläche begünstigt wird. Alternativ können die Mikrorauhigkeiten auch durch ein anderes Verfahren erzeugt werden, beispielsweise durch Ätzen.

In einer weiteren Ausführungsform der Erfindung wird unter einer ersten Ebene von Kavitäten unter der Werkstückoberfläche eine zweite Ebene von Kavitäten erzeugt. Je nach Anwendung können noch weitere Ebenen von Kavitäten erzeugt werden. Dies ermöglicht zum Beispiel eine bessere Verankerung, da der Knochen in ein grösseres Volumen einwachsen kann. Vorteilhaft lässt sich zum Beispiel die unterste Kavitätenschicht mit einer Substanz füllen, die dann kontinuierlich an den Knochen abgegeben wird, der in die obere Schicht einwächst.

Vorteilhaft ist zudem, dass sich das Material durch die Kavitäten gezielt in einzelnen Bereichen schwächen lässt. Dadurch lassen sich beispielsweise Zonen mit elastischen Bereichen schaffen, um einen harmonischen Übergang vom Knochen zum festen Implantat zu erzielen und um das Anwachsen gezielt zu fördern.

Eine weitere Aufgabe der Erfindung ist das Schaffen eines Implantats aus einem metallischen Werkstoff mit einer strukturierten Oberfläche, die ein beschleunigtes Einwachsen von Knochengewebe in die Oberfläche ermöglicht. Diese Aufgabe wird mit einem Implantat mit den Merkmalen von Anspruch 5 gelöst, wobei wenigstens benachbarte Kavitäten durch vorzugsweise spitzwinklige Kanten begrenzt sind.

Durch die Gewölbestruktur wird die Oberfläche des Implantats geschwächt, so dass sich diese unter Belastung leicht verbiegen kann. Diese leichten Deformationsbewegungen führen nun dazu, dass innerhalb der Gewölbe Kräfte entstehen, die das Knochengewebe dazu anregen, die Belastung aufnehmende Strukturen zu bilden.

Das Wachstum wird dabei gerade dort gefördert, wo es am meisten erwünscht ist, nämlich innerhalb der Gewölbestruktur unter der Oberfläche.

Ein weiterer Vorteil besteht darin, dass die Oberflächenstruktur selbstbelüftend ist beim Eindringen von flüssigen oder pastösen Stoffen. Es bilden sich keine Lufteinschlüsse wie z.B. bei Sacklöchern. Dies wirkt sich auch positiv aus bei der Benetzung im Körper mit Blut bei der Implantation. Zudem können in die Vertiefungen gefüllte Wirkstoffe nicht abgestreift werden.

Im Bereich der Kanten wird zudem eine höhere Belastung auf das Knochengewebe erzeugt als in den restlichen Bereichen. Diese höheren Schubspannungen regen das Knochenwachstum zusätzlich an.

Versuche haben gezeigt, dass spitzwinklige Kanten mit einem Kantenwinkel von weniger als 90° sich besonders gut eignen. Selbstverständlich sind aber auch andere Kantenwinkel von mehr als 90° denkbar. In der Praxis werden je nach Konfiguration der Gewölbestruktur Mischformen auftreten, bei denen sich verschiedene Kantenwinkel abwechseln bzw. ineinander übergehen.

Besonders bevorzugt weisen die Kavitäten eine mikrorauhe Oberfläche, insbesondere mit einer Oberflächenrauhigkeit im Bereich von 1 bis 9µm auf, ganz besonders bevorzugt im Bereich von 2.8 bis 6.7µm. Mikrorauhigkeiten in diesem Bereich haben eine positive Wirkung auf Osteoblasten was die Osseointegration der Oberfläche fördert.

Die Kavitäten können kugelkalottenförmige ausgebildet sein. Solche Kavitäten lassen sich relativ einfach mit dem erfindungsgemässen Verfahren herstellen und sie bilden an den Schnittstellen besonders vorteilhafte spitzwinklige Kanten. Die Kavitäten können auch beliebige andere Raumformen aufweisen, beispielsweise Kegel, Pyramiden oder andere beliebige Polyeder. Die Kavitäten kommunizieren dabei mit der Oberfläche, sind also an ihrer Spitze entweder "abgeschnitten" oder weisen einen Kanal, vorzugsweise in Form eines im Querschnitt runden oder polygonalen Schachts, zur Oberfläche auf.

Unter der Oberfläche sind die Kavitäten miteinander verbunden und bilden so eine Gewölbestruktur. In einer besonders bevorzugten Ausführungsform der Erfindung werden die Kavitäten derart angeordnet, dass sie sich unter der Oberfläche teilweise überschneiden, wobei die Anordnung so gewählt wird, dass zwischen den Kavitäten noch genügend Material bestehen bleibt um die zusammenhängende Oberfläche genügend zu stützen. Die Gewölbestruktur kann dabei derart beschaffen sein, dass jede Kavität mit allen benachbarten Kavitäten verbunden ist. Andere Ausführungsformen der Erfindung können vorsehen, dass einzelne Kavitäten nur mit bestimmten anderen Kavitäten verbunden sind. Zum Beispiel können Kavitäten die sich in derselben Reihe oder in einer anderen, beliebigen räumlichen Anordnung zueinander stehen, miteinander verbunden sein.

Das Implantat kann dabei aus jedem geeigneten bioverträglichen Metall bestehen. Bevorzugt besteht das Implantat jedoch aus Titan oder einer Titanlegierung, wie beispielsweise TAV (Ti-6Al-4V) oder TAN (Ti-6Al-7Nb). Das Implantat kann jedoch auch aus anderen in der Medizinaltechnik gebräuchlichen Metallen und/oder Legierungen bestehen, beispielsweise aus rostfreiem Stahl, CoCr Tantal. Weiter kann das Implantat auch aus Gold-, Silber- oder CoCr-Legierungen bestehen sowie in einer besonderen Ausführungsform aus superelastischer Nickel-Titan Legierung.

Wenn das Implantat Kavitäten in Form von Kugelkalotten aufweist, können dabei mindestens ¾ des Kugeldurchmessers unter der Implantatoberfläche liegen. Eine derartige Konfiguration der Oberflächenstrukturen ist besonders vorteilhaft, da die Öffnungen der Kavitäten zur Oberfläche bzw. zum der Öffnungen hin einen spitzen Winkel bilden.

Eine andere Ausführungsform der Erfindung weist Kavitäten auf, die in etwa kubisch ausgebildet sind.

Bevorzugt kann unter einer ersten Ebene von Kavitäten wenigstens eine weitere Ebene von Kavitäten angeordnet sein, die mit den Kavitäten der ersten Ebene in Verbindung stehen. Dabei können die Kavitäten der unteren Ebene so angeordnet werden, dass sie sich mit den Kavitäten der oberen Ebene überschneiden. Alternativ können die Kavitäten beider Ebenen durch Schächte miteinander verbunden sein.

Eine Ausführungsform der Erfindung sieht vor, dass die Kavitäten derart konfiguriert und angeordnet sind, dass eine vorbestimmte Materialfestigkeit unter der Oberfläche erzielbar ist. Damit lässt sich die Festigkeit und Elastizität der Implantatoberfläche je nach Anwendung variieren.

Die Kavitäten können mit einer Substanz, besonders bevorzugt mit einem Wirkstoff gefüllt sein. In Frage kommen dabei verschiedene Wirkstoffe, die das Knochenwachstum fördern, beispielsweise Wachstumsfaktoren. Bevorzugt können Wirkstoffe je nach Indikation und Bedarf intraoperativ durch den Operateur in die Kavitäten eingebracht werden, beispielsweise in Form einer gelartigen Masse oder viskösen Flüssigkeit. Dadurch wird verhindert, dass Einbringen des Implantats die Wirkstoffe abgestreift werden. Die Kavitäten können vorzugsweise auch mit knochenähnlichen mineralischen Substanzen, wie beispielsweise Hydroxylapatit oder β-Tricalciumphosphat, gefüllt werden.

Die Öffnungen können an der Oberfläche ein regelmässiges Muster bilden. Es ist aber auch denkbar, dass die Öffnungen und auch die darunter liegenden Kavitäten unregelmässig angeordnet sind oder dass je nach Anwendungsfall ein regelmässiges Muster in ein unregelmässiges Muster übergeht.

Die Relation zwischen der Grösse der Öffnungen bezogen auf deren lichte Weite und der maximalen Abmessungen der darunter liegenden Kavitäten, kann unterschiedlich ausgestaltet werden. Vorzugsweise sind sowohl die Öffnungen, als auch die darunterliegenden Kavitäten rotationssymmetrisch ausgebildet, wobei der Durchmesser der Kavitäten vorzugsweise wenigstens dem 1,5-fachen des Durchmessers der Öffnungen entspricht.

Weitere Vorteile und Einzelmerkmale der Erfindung ergeben sich aus der nachfolgenden Beschreibung von Ausführungsbeispielen und aus den Zeichnungen. Es zeigen:
- Figur 1:: Eine teilweise geschnittene perspektivische Darstellung einer Ausführungsform der struktu- rierten Oberfläche,
- Figur 2a bis 2d:: eine stark vereinfachte Darstellung eines elektrochemischen Abtragverfahrens,
- Figur 3:: eine schematisierte Darstellung eines Mehrfach- Werkzeuges,
- Figur 4:: eine teilweise geschnittene perspektivische Darstellung einer zweiten Ausführungsform einer strukturierten Oberfläche,
- Figur 5:: einen Querschnitt durch eine kugelkalottenför- mige Gewölbestruktur,
- Figur 6:: einen Querschnitt durch eine tonnenförmige Ge- wölbestruktur,
- Figur 7:: eine Draufsicht auf eine Oberfläche mit unre- gelmässig angeordneten Öffnungen,
- Figur 8:: einen Querschnitt durch eine relativ flache Ge- wölbestruktur,
- Figur 9:: einen Querschnitt durch eine Gewölbestruktur mit zwei Ebenen von Kavitäten, die sich schnei- den,
- Figur 10:: einen Querschnitt durch eine Gewölbestruktur mit zwei Ebenen von Kavitäten, die durch Schächte verbunden sind,
- Figur 11:: eine teilweise geschnittene perspektivische Darstellung einer strukturierten Oberfläche an einem zylindrischen Werkstück, und
- Figur 12:: einen Dünnschnitt durch ein Probeimplantat, das in einem Schafsknochen eingesetzt wurde.

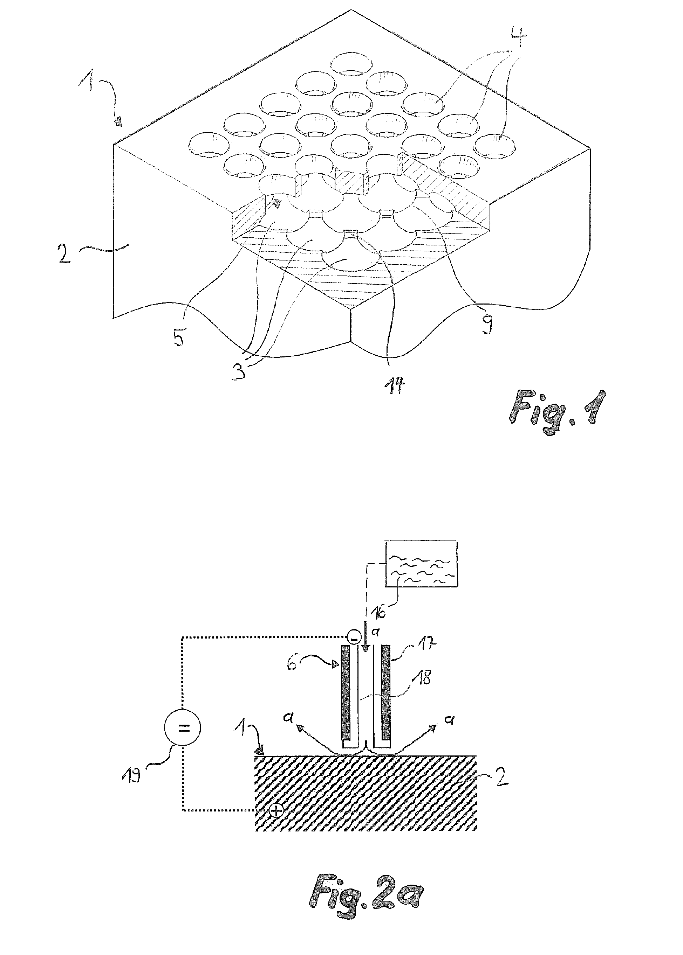

Wie in Figur 1 dargestellt, besteht die strukturiert Oberfläche 1 auf einem Werkstück 2 aus mehreren Kavitäten 3. Dabei ist jede Kavität über eine Öffnung 4 mit der Oberfläche 1 verbunden. Die Kavitäten sind dabei derart angeordnet, dass sie sich unter der Oberfläche überschneiden und so eine zusammenhängende Gewölbestruktur 5 bilden. Die Anordnung ist dabei so gewählt, dass zwischen den Kavitäten noch genügend Material vorhanden ist um Stützpfeiler 14 für die Oberfläche auszubilden. Das verwendete Herstellungsverfahren ermöglicht es, dass sich an den Überschneidungsflächen der Kavitäten spitzwinklige Kanten 9 bilden. Vorzugsweise fehlen in gewissen Bereichen die Stützpfeiler 14. Dadurch kann in diesen Bereichen eine höhere Biegbarkeit erreicht werden. Die Kavitäten können beispielsweise bei einem Durchmesser von 15 bis 20mm Öffnungen mit einem Radius von 4 bis 8mm aufweisen.

Figuren 2a bis 2d zeigen schematisch das Herstellungsverfahren einer Kavität. Wie in Figur 2a gezeigt, wird ein Werkzeug 6 in die Nähe der Oberfläche 1 des Werkstücks 2 gebracht. Durch einen Kanal 18 im Werkzeug 6 fliesst in Pfeilrichtung a eine Elektrolytlösung 16 auf die Oberfläche 1. Das Werkstück 2 wird an die Anode eines Gleichstromgenerators 19 angeschlossen und das Werkzeug 6 wird an die Kathode angeschlossen. Das Werkzeug 6 ist auf der Aussenseite teilweise durch eine Isolationsschicht 17 elektrisch isoliert. Die Elektrolytlösung 16 leitet den Strom im Spalt zwischen dem positiv polarisierten Werkstück 2 und dem negativ polarisierten Werkzeug 6. Bevorzugt werden Natriumnitrat-oder Natriumchloridlösungen als Elektrolyt verwendet. Jedoch kann auch jede andere geeignete Elektrolytlösung verwendet werden. Durch Anlegen einer Spannung wird nun an der Annode das Metall des Werkstücks 2 unter Abgabe von Elektronen ionisiert. Diese Metallionen gehen in die Elektrolytlösung über. Dadurch entsteht ein Materialabtrag an der Oberfläche des Werkstücks 2.

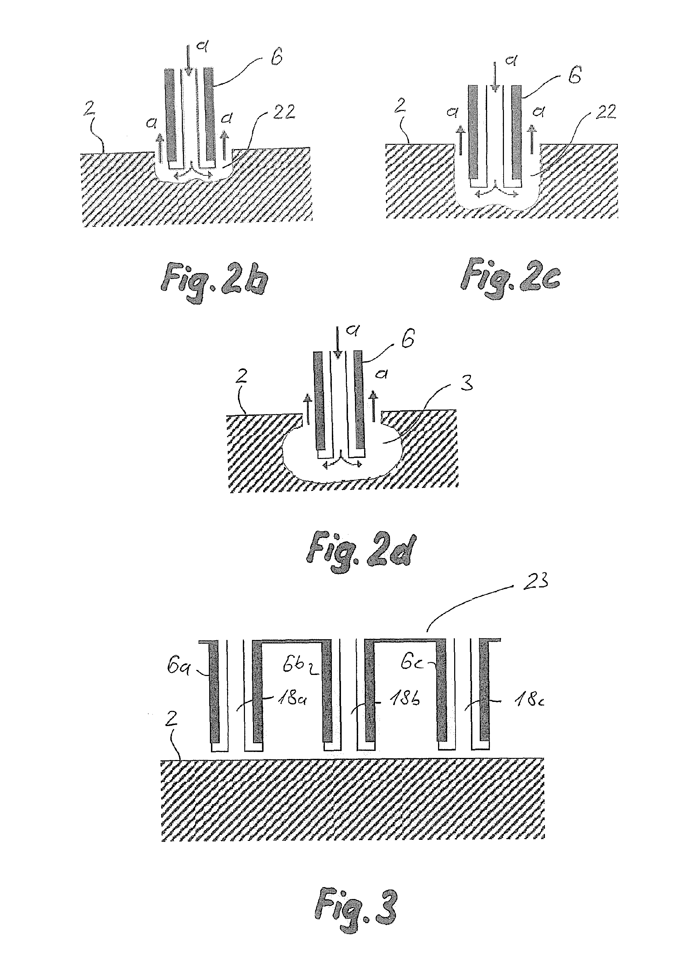

Durch Vorschub des Werkzeugs lässt sich ein beliebig tiefes Sackloch 22 abtragen, wie exemplarisch in Figur 2b gezeigt. Bevorzugt ist das Werkzeug derart isoliert, dass nur dessen Spitze elektrisch mit der Elektrolytlösung in Kontakt steht. Dadurch lässt sich vermeiden, dass das Sackloch durch stetiges Abtragen im Bereich zwischen der Oberfläche und der Spitze verbreitert wird. Ist das Werkzeug tief genug unter der Oberfläche, wird der Vorschub angehalten. Durch den anhaltenden Elektrolytfluss wird nach und nach im Bereich der Werkzeugspitze Material abgetragen, wie in den Figuren 2c und 2d gezeigt. Dadurch bildet und verbreitert sich die Kavität 3 im Inneren des Werkstücks. Durch geschickte Wahl der Parameter wie Vorschubgeschwindigkeit, Spüldruck oder Spannung lassen sich Kavitäten mit verschiedenen Raumformen erzeugen.

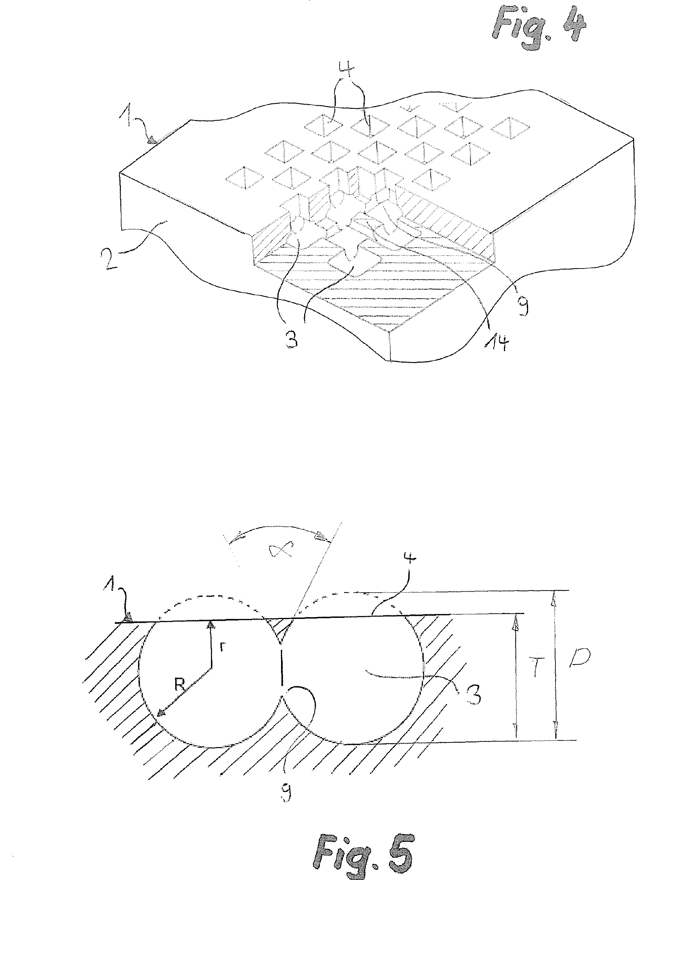

Zur effizienten Herstellung einer strukturierten Oberfläche wird besonders bevorzugt ein Mehrfachwerkzeug 23 verwendet, wie es vereinfacht in Figur 3 dargestellt ist. Das Mehrfachwerkzeug weist dabei mehrere Einzelwerkzeuge 6a, 6b und 6c auf, die fest miteinander verbunden sind und von denen jedes über einen separaten Kanal 18a, 18b und 18c verfügt. Die Anordnung entspricht dabei ersichtlicherweise dem gewünschten Oberflächenmuster. Figur 4 zeigt eine weitere exemplarische Ausführungsform der erfindungsgemässen strukturierten Oberfläche. Bei dieser Ausführung sind die Kavitäten 3 sowie deren Öffnungen 4 auf der Oberfläche 1 quaderförmig. Eine solche Form lässt sich beispielsweise durch ein entsprechend geformtes Werkzeug erzeugen. Bei dieser Ausführungsform sind die Kanten 9 im Querschnitt eher etwa rechtwinklig ausgebildet.

Einen Schnitt durch eine weitere Ausführungsform der Erfindung zeigt Figur 5. Die Kavitäten 3 haben hier die Form von Kugelkalotten. Die räumliche Anordnung der einzelnen Kalotten ist so gewählt, dass sich jeweils benachbarte Kalotten überschneiden und so eine Gewölbestruktur unter der Oberfläche bilden. Die Kugelkalotten haben dabei einen Durchmesser D, wobei die Tiefe T in der Oberfläche so gewählt ist, dass diese wenigstens ¾ des Durchmessers der Kugel beträgt. Die Distanz r vom Zentrum der Kugel bis zur Oberfläche 1 ist also mindestens halb so lang wie der Kugelradius R. Ersichtlicherweise bilden sich so auch scharfe, spitzwinklige Kanten, welche einerseits die Öffnungen 4 an der Oberfläche begrenzen und andererseits auch die benachbarten, miteinander verbundenen Kavitäten 3. Im Querschnitt haben die Kanten 9 einen Kantenwinkel α von deutlich weniger als 90°.

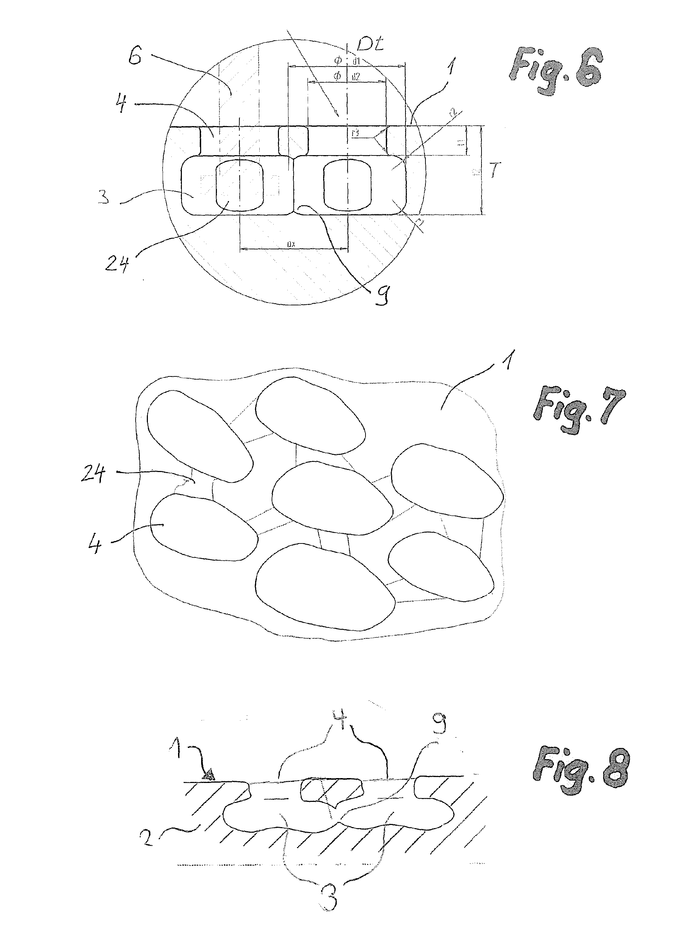

Figur 6 zeigt einen vergrösserten Querschnitt durch eine Gewölbestruktur, bei der die einzelnen Kavitäten etwa tonnenartig ausgebildet sind, wobei die einzelnen Tonnen einander schneiden und auf diese Weise Durchbrüche 24 bilden. Lediglich angedeutet ist hier auf einer Seite das Werkzeug 6, dessen Aussendurchmesser am unteren Ende etwa dem Innendurchmesser der Öffnungen 4 entspricht. Die Kanten 9, welche die Durchbrüche 24 begrenzen, können hier einen Kantenwinkel von mehr als 90° aufweisen. Die Tiefe T der gesamten Struktur kann hier ca. 0,9mm betragen und zwar bei einem Durchmesser Dt der Tonnen von 1,2mm.

Figur 7 zeigt eine Draufsicht auf eine Oberfläche 1 mit völlig unregelmässig angeordneten Öffnungen 4, die unter der Oberfläche mit Durchbrüchen 24 miteinander verbunden sind.

Gemäss Figur 8 sind die Kavitäten 3 relativ flach ausgebildet und sie verfügen nicht über zylindrische Innenwände wie beim Ausführungsbeispiel gemäss Figur 6. Wie dargestellt haben die Kavitäten 3 eine eher elliposidale Form. Solche Kavitäten haben den Vorteil, dass eine dickere Materialschicht zwischen den Kavitäten 3 und der Oberfläche 1 vorhanden ist. Ausserdem lassen sich mit dieser Struktur grössere Abstände zwischen den Öffnungen 4 erreichen.

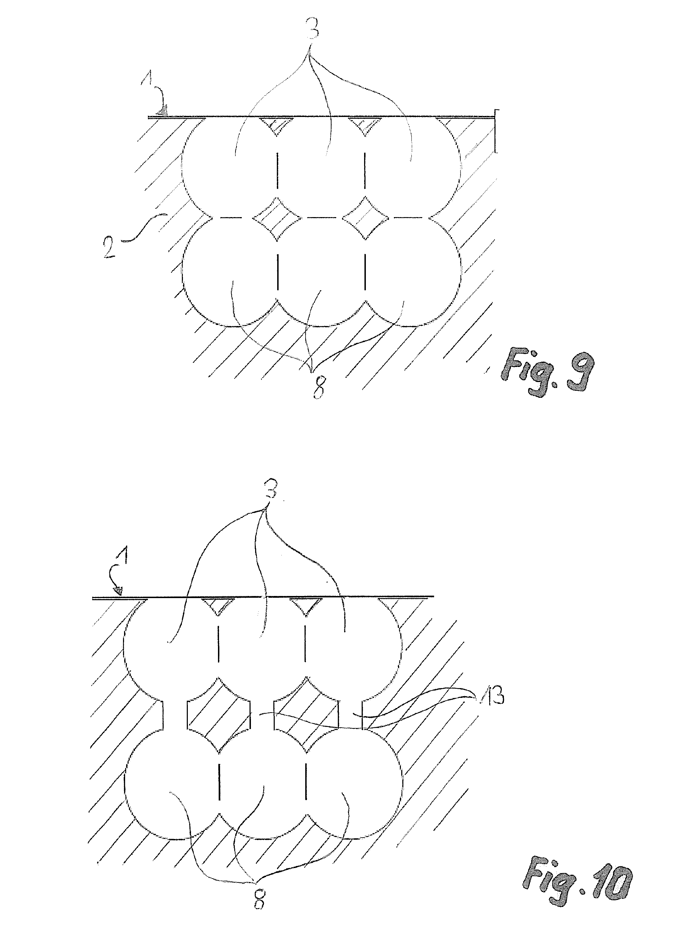

Gemäss Figur 9 liegt unter einer ersten Ebene von Kavitäten 3 eine zweite Ebene von Kavitäten 8. Die Kavitäten können räumlich derart angeordnet werden, dass die Kavitäten der zweiten Ebene 8 sich mit den Kavitäten der ersten Ebene 3 überschneiden, wie in Figur 9 gezeigt. Alternativ können die Kavitäten der zweiten Ebene 8 durch Verbindungskanäle 13 mit den Kavitäten der ersten Ebene verbunden sein, wie in Figur 10 dargestellt. Es wäre ausserdem denkbar, dass sich unter der zweiten Ebene noch weitere Ebenen mit Kavitäten anlegen lassen. Die Kavitäten müssen auf verschiedenen Ebenen auch nicht zwingendermassen die gleiche Konfiguration aufweisen.

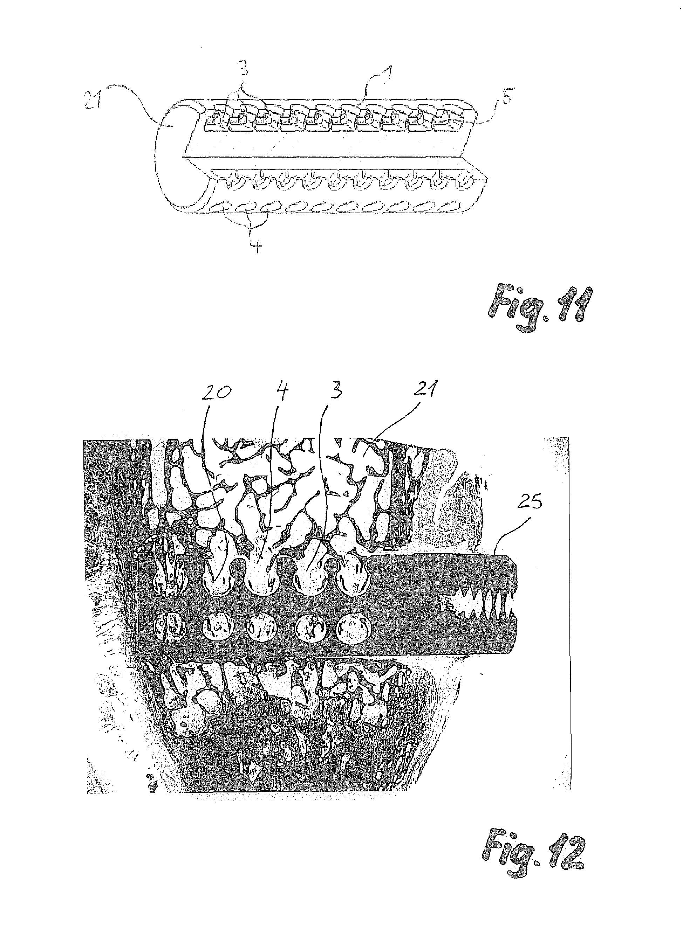

Figur 11 zeigt einen Teilschnitt eines zylindrischen Implantats 21 mit einer strukturierten Oberfläche. Die Öffnungen 4 sind bei diesem Beispiel reihenweise angeordnet, wobei sich die Kavitäten 3 derselben Reihe jeweils überschneiden. Natürlich liesse sich auch ein Implantat herstellen, bei dem sich Kavitäten verschiedener Reihen überschneiden. Ausserdem lassen sich erfindungsgemässe Oberflächen an Werkstücken von beliebiger Konfiguration anbringen, also beispielsweise auch an konkav oder konvex gewölbten Oberflächen.

Figur 12 zeigt ein Dünnschnittbild eines an einem Schenkelknochen 21 eines Schafes implantierten Versuchsimplantats 25 nach 8 Wochen. Die Kavitäten 3 bei diesem Versuch waren 1.5mm tief bei einem Durchmesser von 1.9mm. Die Öffnungen 4 zur Oberfläche hin wiesen einen Durchmesser von 1.4mm auf. Das Implantat wurde mit dem erfindungsgemässen Verfahren aus Titan Grade 5 gefertigt. Schön zu sehen ist wie Knochengewebe 20 in die Kavitäten 3 einwächst.

Auf Grund der gewählten Schnittebene sind hier die Durchbrüche zwischen den einzelnen Kavitäten gerade nicht sichtbar. Auf Grund der nur wenige zehntel Millimeter dicken Schnittprobe wären sonst die verbleibenden Stützenköpfe weggebrochen.

## Patentansprüche

1. Verfahren zur Herstellung einer strukturierten Oberfläche (1) an einem Werkstück (2) aus einem metallischen Werkstoff, insbesondere an einem Implantat, wobei die Oberfläche mit einer Mehrzahl von sich gegen das Innere des Werkstücks erweiternde Kavitäten (3) versehen ist, die zur Oberfläche hin separate Öffnungen (4) aufweisen und die zur Bildung einer Gewölbestruktur (5) unter der Oberfläche miteinander verbunden sind, **dadurch gekennzeichnet, dass** die Kavitäten durch ein elektrochemisches Abtragverfahren erzeugt werden.

2. Verfahren nach Anspruch 1, **dadurch gekennzeichnet, dass** jeweils mehrere Kavitäten (3) mit einem mehrere Kathoden umfassenden Mehrfachwerkzeug gleichzeitig erzeugt werden.

3. Verfahren nach einem der Ansprüche 1 oder 2, **dadurch gekennzeichnet, dass** nach der Erzeugung der Kavitäten (3) als Grobstruktur die Parameter des Abtragprozesses, insbesondere die Stromdichte und/oder der Spüleffekt zwischen Werkstück und Werkzeug mit Hilfe des Elektrolyts derart verändert werden, dass die Kavitäten eine mikrorauhe Oberfläche, insbesondere mit einer Oberflächenrauhigkeit im Bereich von 1 bis 9µm erhalten.

4. Verfahren nach einem der Ansprüche 1 bis 3, **dadurch gekennzeichnet, dass** unter einer ersten Ebene von Kavitäten (3) unter der Werkstückoberfläche eine zweite Ebene von Kavitäten (8) erzeugt wird.

5. Implantat aus einem metallischen Werkstoff mit einer strukturierten Oberfläche (1), welche eine Mehrzahl von sich gegen das Innere des Implantats erstreckende Kavitäten (3) aufweist, die zur Oberfläche hin separate Öffnungen (4) bilden und die zur Bildung einer Gewölbestruktur (5) unter der Oberfläche miteinander verbunden sind, **dadurch gekennzeichnet, dass** die Kavitäten (3) derart durch ein elektrochemisches Abtragverfahren erzeugt werden, dass wenigstens benachbarte Kavitäten (3) durch vorzugsweise spitzwinklige Kanten (9) begrenzt sind.

6. Implantat nach Anspruch 5, **dadurch gekennzeichnet, dass** die Kavitäten (3) eine mikrorauhe Oberfläche, insbesondere mit einer Oberflächenrauhigkeit im Bereich von 1 bis 9 µm aufweisen.

7. Implantat nach einem der Ansprüche 5 oder 6, **dadurch gekennzeichnet, dass** die Kavitäten (3) kugelkalottenförmig ausgebildet sind.

8. Implantat nach Anspruch 7, **dadurch gekennzeichnet, dass** mindestens 75% des Durchmessers der Kavitäten (3) unter der Oberfläche liegt.

9. Implantat nach einem der Ansprüche 5 oder 6, **dadurch gekennzeichnet, dass** die Kavitäten (3) etwa kubisch ausgebildet sind.

10. Implantat nach einem der Ansprüche 5 bis 9, **dadurch gekennzeichnet, dass** unter einer ersten Ebene von Kavitäten (3) wenigstens eine weitere Ebene von Kavitäten (8) angeordnet ist, die mit den Kavitäten (3) der ersten Ebene in Verbindung stehen.

11. Implantat nach einem der Ansprüche 5 bis 10, **dadurch gekennzeichnet, dass** die Kavitäten (3;8) derart konfiguriert und angeordnet sind, dass eine vorbestimmte Materialsteifigkeit unter der Oberfläche erzielbar ist.

12. Implantat insbesondere nach einem der Ansprüche 5 bis 11, **dadurch gekennzeichnet, dass** die Kavitäten (3;8) mindestens teilweise mit einem Wirkstoff gefüllt sind.

13. Implantat nach einem der Ansprüche 5 bis 12, **dadurch gekennzeichnet, dass** die Öffnungen an der Oberfläche ein regelmässiges Muster bilden.

14. Implantat nach einem der Ansprüche 5 bis 13, **dadurch gekennzeichnet, dass** die Öffnungen und die darunter liegenden Kavitäten rotationssymmetrisch ausgebildet sind und dass vorzugsweise der Durchmesser der Kavitäten wenigstens dem 1,5-fachen des Durchmessers der Öffnungen entspricht.
